(19) Europäisches Patentamt
European Patent Office
Office européen des brevets

(11) Veröffentlichungsnummer: **0 157 740**
**B1**

(12) # EUROPÄISCHE PATENTSCHRIFT

(45) Veröffentlichungstag der Patentschrift: **27.06.90**

(21) Anmeldenummer: **85810139.7**

(22) Anmeldetag: **29.03.85**

(51) Int. Cl.$^5$: **C 07 D 303/32,**
C 07 C 49/784, C 07 C 49/782,
C 08 G 59/24, C 08 G 59/32,
C 07 C 49/83, C 07 C 49/84

(54) **Glycidyloxydiketone.**

(30) Priorität: **04.04.84 CH 1693/84**

(43) Veröffentlichungstag der Anmeldung:
**09.10.85 Patentblatt 85/41**

(45) Bekanntmachung des Hinweises auf die Patenterteilung:
**27.06.90 Patentblatt 90/26**

(84) Benannte Vertragsstaaten:
**CH DE FR GB IT LI NL**

(56) Entgegenhaltungen:
**FR-A-2 330 709**
**GB-A-1 276 145**
**GB-A-1 276 146**
**GB-A-2 098 868**
**US-A-3 522 210**

(73) Patentinhaber: **CIBA-GEIGY AG**
**Klybeckstrasse 141**
**CH-4002 Basel (CH)**

(72) Erfinder: **Darms, Roland, Dr.**
**Kleinfeldweg 3**
**CH-4106 Therwil (CH)**
Erfinder: **Monnier, Charles E., Dr.**
**Ch. du Verger 16**
**CH-1752 Villars-sur-Glâne (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft neue Di-, Tri- oder Tetraglycidyloxydiketone, ein Verfahren zu deren Herstellung sowie deren Verwendung zur Herstellung von vernetzten ausgehärteten Produkten.

Epoxidharze finden Anwendung in zahlreichen Gebieten, z.B. als Klebstoffe, Lacke, Pressmassen, Isolatoren und Verbundwerkstoffe, und im Handel wird eine Vielfalt chemisch verschiedener Epoxidharze angeboten. Die üblicherweise verwendeten Epoxidharze sind von einem Bisphenol, einer Dicarbonsäure oder von einem Diamin und Epichlorhydrin abgeleitete Glycidylderivate.

Auch Glycidyloxy-substituierte Benzophenone und deren Verwendung als Epoxidharze sind bekannt; vgl. z.B. A. L. Cupples et al. [Advan. Chem. Ser. *92*, 173—207 (1970)] und von E. S. Dzhavadyan et al. [Polymer Bulletin *4*, 479—485 (1981)].

Aus der US Patentschrift 2,922,777 ist weiterhin bekannt, dass man u.a. ortho-Hydroxyglycidyloxybenzophenone sowie 1,3-Bis(4'-glycidyloxy-2'-hydroxybenzoyl)benzol als Licht- bzw. Wärmeschutzmittel für Polyolefine einsetzen kann.

Die GB—A—1 276 146 und die GB—A—1 276 145 beschreiben substituierte 1,3-Bis-(2'-hydroxybenzoyl)benzole und deren Verwendung als Lichtschutzmittel in organischen Materialien.

Die GB—A—2 098 868 beschreibt kosmetische Stoffgemische, welche als UV-Absorber substituierte 2-Hydroxydibenzoylmethane enthalten.

Die FR—A—2 330 709 beschreibt ungesättigte photopholymerisierbare glycidylgruppenhaltige Ketone und deren Anwendung für die Herstellung von Polymeren durch Bestrahlung, insbesondere für die Herstellung von Abbildungen.

Die US Patentschrift 3,522,210 beschreibt härtbare Polyglycidylether bestimmter zweiwertiger Phenole, bei denen jeweils zwei Phenolreste über Ether sauerstoffatome an einen flexibilen zweitwertigen Rest gebunden sind.

Gegenstand der Erfindung sind neue Glycidyloxydiketone der Formel I

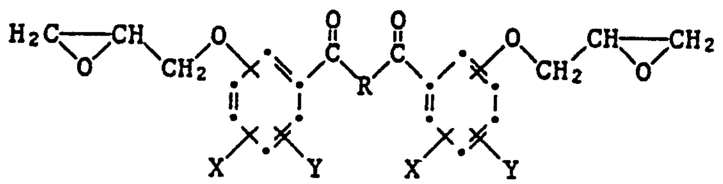

(I),

worin die Substituenten X unabhängig voneinander Wassersstoff, Alkyl mit 1 bit 6 C-Atomen, Glycidyloxy oder Alkoxy mit 1 bis 6 C-Atomen bedeuten, die Substituenten Y unabhängig voneinander Wasserstoff oder Alkyl mit 1 bis 6 C-Atomen bedeuten und R für eine direkte Bindung, eine Gruppe der Formel $—C_mH_{2m}—$, $—C_nH_{2n-2}—$ oder $—C_nH_{2n-4}—$ mit m=1—12 und N=2—12, einen zweiwertigen, gegebenenfalls ungesättigten cycloaliphatischen Rest mit bis zu 14 C-Atomen, einen zweiwertigen araliphatischen Rest mit bis zu 14 C-Atomen, einen zweiwertigen $C_6—C_{12}$ aromatischen Rest oder eine Gruppe der Formel II

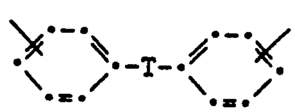

(II),

worin T eine direkte Bindung, Methylen, Isopropyliden, O, S, NH, CO oder $SO_2$ bedeutet, steht.

Die Verbindungen der Formel I können z.B. dadurch erhalten werden, dass man eine Verbindung der Formel III

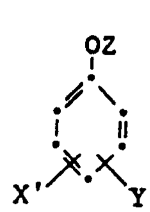

(III)

oder ein Gemisch verschiedener Verbindungen der Formel III, worin Z Wasserstoff oder Alkyl mit 1 bis 6 C-Atomen und X' Wasserstoff, Alkyl mit 1 bis 6 C-Atomen, Hydroxy oder Alkoxy mit 1 bis 6-Atomen darstellen und für Y das oben Angegebene gilt, in Anwesenheit eines Friedel-Crafts-Katalysators mit einer Verbindung der Formel IV

Cl—CO—R—CO—Cl (IV)

worin R die oben angegebene Bedeutung hat, in eine Verbindung der Formel V

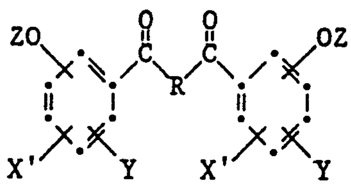

(V)

überführt, die Verbindungen der Formel V gegebenenfalls O-dealkyliert (d.h. in eine Verbindung der Formel V mit Z=H und X'=H, Alkyl mit 1 bis 6 C-Atomen oder Hydroxy überführt) und anschliessend mit einem Epihalogenhydrin umsetzt. Vor oder nach der Reaktion mit dem Epihalogenhydrin kann gegebenenfalls eine O-Alkylierung mit einem $C_1$—$C_6$-Alkylhalogenid durchgeführt werden.

Alkylsubstituenten X und Y mit 1 bis 6 C-Atomen können geradkettig oder verzweigt sein. Es kommen beispielsweise Methyl, Ethyl, n- und iso-Propyl sowie die verschiedenen Butyl-, Pentyl- und Hexyl-Isomeren in Frage. Dasselbe gilt für die entsprechenden O-Alkylreste der Alkoxysubstituenten mit 1 bis 6 C-Atomen. Im allgemeinen werden geradkettige Alkyl- oder Alkoxyreste bevorzugt, besonders Methyl, Ethyl, Methoxy und Ethoxy.

Die Brückenglieder R der Formeln $—C_mH_{2m}—$, $—C_nH_{2n-2}—$ und $—C_nH_{2n-4}—$ können geradkettige und verzweigte zweiwertige Alkylen-, Alkenylen-, Alkadienylen- oder Alkynylenreste sein, wie z.B. 1,4-, 1,3- oder 1,2-Butylen, 1,6- oder 1,3-Hexylen, 1,8-, 2,7- oder 3,6-Octylen, 1,2-Ethenylen, 1,4-But-2-enylen, 1,6-Hexa-2,4-dienylen und 1,6-Hex-3-inylen.

Als Beispiele für gegebenenfalls ungesättigte cycloaliphatische zweiwertige Reste R seien genannt: 1,2- oder 1,3-Cyclopentylen, 1,2-, 1,3- oder 1,4-Cyclohexylen, 1,2-, 1,3-, 1,4-, 3,4- und 3,5- 3,5-Cyclopentenylen, der 4,4'-Bis(cyclohexylen)methan- und Dimethylencyclohexanrest, 1,2- und 3,6-Cyclohexenylen.

Zweiwertige araliphatische Reste R sind beispielsweise die Dimethylenbenzol-, Dimethylentoluol- und Dimethylennaphthalinreste.

Zweiwertige aromatische Rest R mit 6 bis 12 C-Atomen sind beispielsweise 1,2-, 1,3- oder 1,4-Phenylengruppen, Toluylengruppen und Naphthylengruppen, wie die 2,6-, 1,4- und 1,5-Naphthylengruppe.

Gegenstand der Erfindung sind auch Verbindungen der Formel V, worin R, Z, Y und X' die oben angegebene Bedeutung haben, mit der Massgabe, dass R nicht 1,3-Phenylen oder $C_1$—$C_5$-Alkyliden ist.

Bevorzugte Verbindungen der Formel I sind solche, worin die Substituenten X sowie die Substituenten Y jeweils gleich sind, die Substituenten X und Y sowie die Glycidylgruppen jeweils in der gleichen Stellung in Bezug auf die Carbonylgruppe sind und R für eine Gruppe der Formel $—C_mH_{2m}—$ mit m gleich 1 bis 8 oder Phenylen steht.

Besonders bevorzugt sind Verbindungen der Formel I, worin die Substituenten X jeweils Wasserstoff, Alkyl mit 1 bis 4 C-Atomen oder Alkoxy mit 1—4 C-Atomen und die Substituenten Y jeweils Wasserstoff oder Alkyl mit 1 bis 4 C-Atomen bedeuten, und die Glycidyloxygruppen jeweils in der para-Stellung zu den Carbonylgruppen gebunden sind.

Ganz besonders bevorzugt sind Verbindungen der Formel I, worin die Substituenten X und Y jeweils gleich sind und Methyl oder insbesondere Wasserstoff bedeuten und R für 1,4-Phenylen, 1,3-Phenylen, 1,4-Butylen oder 1,8-Octylen steht.

Weiterhin sind Verbindungen der Formel I bevorzugt, worin die Substituenten X jeweils eine Glycidyloxygruppe und die Substituenten Y jeweils Wasserstoff oder eine Alkylgruppe mit 1 bis 4 C-Atomen darstellen, und die Glycidyloxygruppen jeweils in der ortho- und der para-Stellung zu den Carbonylgruppen gebunden sind. Darunter sind besonders die Verbindungen bevorzugt, worin die Substituenten Y jeweils Wasserstoff sind und R für 1,4-Butylen und insbesondere für 1,3-Phenylen oder 1,4-Phenylen steht.

Die Ausgangsprodukte der Formeln III und IV sind an sich bekannt oder können auf an sich bekannte Weise hergestellt werden.

Die Herstellung der Verbindungen der Formel V, d.h. der bei der Synthese der erfindungsgemässen Verbindungen der Formel I anfallenden Zwischenprodukte, kann analog den in der US-Patentschriften 3,821,310 und 4,276,226 beschriebenen Verfahren durchgeführt werden.

Bei der Friedel-Crafts-Umsetzung der Verbindungen der Formel III mit den Dicarbonsäuredichloriden der Formel IV können Alkoxygruppen OZ und/oder X' in Hydroxygruppen umgewandelt werden, besonders wenn sie im Reaktionsprodukt der Formel V in ortho-Stellung zur Carbonylgruppe stehen. Falls erwünscht, können noch vorhandene Alkoxygruppen anschliessend durch weiteres Erhitzen in Anwesenheit des Friedel-Crafts-Katalysators entalkyliert werden.

Die erfindungsgemässen Endprodukte der Formel I erhält man schliesslich durch Umsetzung von Verbindungen de Formel V mit einem Epihalogenhydrin, bevorzugt Epichlorhydrin, in Gegenwart einer Base und gegebenenfalls eines Katalysators, Verbindungen der Formel V, worin OZ und/oder X' eine Hydroxygruppe bedeuten, werden vor oder nach der Reaktion mit dem Epihalogenhydrin zur Einführung von Alkoxygruppen mit $C_1$—$_6$-Alkylhalogeniden umgesetzt. Dabei hängt die jeweilige Reaktionsreihenfolge

vom gewünschten Produkt ab. Falls beide Substituenten OZ und X' der Verbindung V Hydroxygruppen sind, findet die erste Alkylierung bzw. Glycidylierung im allgemeinen am OH in der para- oder meta-Stellung zur Carbonylgruppe und die zweite Alkylierung bzw. Glycidylierung am OH in der ortho-Stellung zur Carbonylgruppe statt.

Als Friedel-Crafts-Katalysator für die Umsetzung von Verbindungen der Formeln III und IV sowie für die allfällige Dealkylierung kann beispielsweise Aluminiumtrichlorid verwendet werden.

Die Dealkylierung bzw. Alkylierung von Verbindungen der Formel V kann wie in der US Patentschrift 3,821,310 beschrieben vorgenommen werden.

Die Glycidylierung von Verbindungen der Formel V wird auf an sich bekannte Weise durchgeführt, indem man sie in Anwesenheit einer Base, beispielsweise Natriumhydroxid, und gegebenenfalls eines Katalysators, wie z.B. Tetramethylammoniumchlorid, beir erhöhter Temperatur (ca. 50—150°C) mit einem Epihalogenhydrin, besonders Epichlorhydrin, umsetzt.

Die erfindungsgemässen Verbindungen der Formel I können auf übliche Weise isoliert und gereinigt werden, beispielsweise durch Extraktion oder Umkristallisieren aus geeigneten Lösungsmitteln, wie z.B. aus Aceton oder Methylcellosolve/Wasser, oder durch Filtration an Kieselgel mit geeigneten Lösungsmitteln, beispielsweise Toluol/Ethanol.

Die erfindungsgemässen Verbindungen der Formel I eignen sich als Epoxidharze zur Herstellung von vernetzten Produkten.

Die vorliegende Anmeldung betrifft somit auch härtbare Gemische, die sich beispielsweise zur Herstellung von Formkörpern, Imprägnierungen, Ueberzügen, Verklebungen und insbesondere von Verbundwerkstoffen, Matrixharzen oder Sinterpulverlacken eignen. Sie sind dadurch gekennzeichnet, dass sie

a) eine Verbindung der Formel I und

b) einen Härter und/oder einen Härtungskatalysator für Epoxidharze enthalten.

Als Beispiele für Härtungsmittel seien die gebräuchlichen Härtungsmittel für Epoxidharze genannt, einschliesslich der aliphatischen, cycloaliphatischen, aromatischen und heterocyclischen Amine, wie Bis(4-aminophenyl)methan, Anilin-Formaldehyd-Harz, Bis(4-aminophenyl)sulfon, Propan-1,3-diamin, Hexamethylendiamin, Diethylentriamin, Triethylentetramin, 2,2,4-Trimethylhexan-1,6-diamin, m-Xylylendiamin, Bis(4-aminocyclohexyl)methan, 2,2-Bis(4-aminocyclohexyl)propan und 3-Aminomethyl-3,5,5-trimethylcyclohexylamin (Isophorondiamin), Polyaminoamide, wie beispielsweise solche aus aliphatischen Polyaminen und dimerisierten oder trimerisierten Fettsäuren, Polyphenole, wie Resorcin, Hydrochinon, 2,2-Bis(4-hydroxyphenyl)propan und Phenol-Aldehyd-Harze, Polythiole, wie die im Handel unter der Bezeichnung "Thiokole" erhältlichen Polythiole, Polycarbonsäuren und ihre Anhydride, wie zum Beispiel Phthalsäureanhydrid, Tetrahydrophthalsäureanhydrid, Hexahydrophthalsäureanhydrid, Hexachlorendomethylentetrahydrophthalsäureanhydrid, Pyromellitsäuredianhydrid, Benzophenon-3,3,4',4'-tetracarbonsäuredianhydrid, die Säuren der zuvorgenannten Anhydride sowie auch Isophthalsäure und Terephthalsäure. Es können auch katalytisch wirdkende Härtungsmittel verwendet werden, wie beispielsweise Zinnsalze von Alkansäuren (zum Beispiel Zinnoctanoat), Friedel-Crafts-Katalysatoren, wie Bortrifluorid und Bortrichlorid und ihre Komplexe und Chelate, die durch Umsetzung mit Bortrifluorid mit zum Beispiel 1,3-Diketonen erhalten werden.

Die Menge des eingesetzten Härtungsmittels richtet sich nach der chemischen Natur des Härtungsmittels und nach den gewünschten Eigenschaften der härtbaren Mischung und des gehärteten Produktes. Die maximale Menge kann leicht ermittelt werden. Wenn das Härtungsmittel ein Amin ist, werden normalerweise 0,75 bis 1,25 Aequivalente Aminwasserstoff pro 1 Epoxidäquivalent eingesetzt. Wenn Polycarbonsäuren oder ihre Anhydride eingesetzt werden, verwendet man gewöhnlich 0,4 bis 1,1 Aequivalente Carboxylgruppe bzw. Anhydridgruppe pro 1 Aequivalent Epoxidgruppe. Bei der Verwendung von Polyphenolen als Härtungsmittel setzt man zweckmassig 0,75 bis 1,25 phenolische Hydroxylgruppen pro 1 Epoxidäquivalent ein.

Katalytisch wirkende Härtungsmittel werden allgemein in Mengen von 1 bis 40 Gewichtsteilen pro 100 Gewichtsteile Epoxidharz eingesetzt.

Gewünschtenfalls kann man den härtbaren Gemischen zur Herabsetzung der Viskosität aktive Verdünner, wie z.B. Styroloxid, Butylglycidylether, 2,2,4-Trimethylpentylglycidylether, Phenylglycidylether, Kresylglycidylether oder Glycidylester von synthetischen, hochverzweigten, in der Hauptsache tertiären aliphatischen Monocarbonsäuren, zusetzen.

Man kann bei der Härtung ausserdem Härtungsbeschleuniger einsetzen; solche Beschleuniger sind z.B. tertiäre Amine, deren Salze oder quaternäre Ammoniumverbindungen, z.B. Benzyldimethylamin, 2,4,6-Tris(dimethylaminomethyl)phenol, 1-Methylimidazol, 2-Ethyl-4-methylimidazol, 4-Aminopyridin, Tripentylammoniumphenolat; oder Alkalimetallalkoholate, wie z.B. Na-Alkoholate von 2,4-Dihydroxy-3-hydroxymethylpentan. Die Härtung der erfindungsgemässen Mischungen wird zweckmässig im Temperaturintervall von 50°C bis 300°C, bevorzugt von 80—250°C, durchgeführt.

Man kann die Härtung in bekannter Weise auch zwei- oder mehrstufig durchführen, wobei die erste Härtungsstufe bei niedriger Temperatur und die Nachhärtung bei höherer Temperatur durchgeführt wird.

Die Härtung kann gewünschtenfalls auch derart in 2 Stufen erfolgen, dass die Härtungsreaktion zunächst vorzeitig abgebrochen bzw. die erste Stufe bei wenig erhöhter Temperatur durchgeführt wird, wobei ein noch schmelzbares und/oder lösliches, härtbares Vorkondensat (sogenannte "B-Stufe") aus der

Epoxy-Komponente (a) und dem Härter (b) erhalten wird. Ein derartiges Vorkondensat kann z.B. zur Herstellung von "Prepregs", Pressmassen oder Sinterpulvern dienen.

Der Ausdruck "Härten", wie er hier gebraucht wird, bedeutet die Umwandlung der löslichen, entweder flüssigen oder schmelzbaren Polyepoxide in feste, unlösliche und unschmelzbare, dreidimensional vernetzte Produkte bzw. Werkstoffe, und zwar in der Regel unter gleichzeitiger Formgebung zu Formkörpern, wie Giesskörpern, Presskörpern und Schichtstoffen, zu Imprägnierungen, Beschichtungen, Lackfilmen oder Verklebungen.

Die vorliegenden härtbaren Gemische können ferner geeignete Weichmacher, wie Dibutylphthalat, Dioctylphthalat oder Trikresylphthalat, enthälten.

Schliesslich können die härtbaren Gemische vor der Härtung in irgendeiner Phase mit Streck-, Füll- und Verstärkungsmitteln, wie beispielsweise Steinkohlenteer, Bitumen, Textilfasern, Glasfasern, Asbestfasern, Borfasern, Kohlenstoff-Fasern, mineralsichen Silikaten, Glimmer, Quarzmehl, Aluminiumoxidhydrat, Bentoniten, Kaolin, Kieselsäureaerogel oder Metallpulvern, z.B. Aluminiumpulver oder Eisenpulver, ferner mit Pigmenten und Farbstoffen, wie Russ, Oxidfarben, Titandioxid u.a. versetzt werden. Man kann den härtbaren Gemischen ferner auch andere übliche Zusätze. z.B. Flammschutzmittel, wie Antimontrioxide, Thixotropiemittel, Verlaufmittel ("flow control agents"), wie Silicone, Wachse oder Stearate (welche zum Teil auch als Formtrennmittel Anwendung finden), zusetzen.

Die Herstellung der vorliegenden härtbaren Mischungen kann in üblicher Weise mit Hilfe bekannter Mischaggregate (Rührer, Kneter, Walzen etc.) erfolgen.

Die vorliegenden härtbaren Epoxidharzmischungen finden ihren Einsatz vor allem auf den Gebieten des Oberflächenschutzes, der Elektrotechnik, der Laminierverfahren und im Bauwesen. Sie können in jeweils dem speziellen Anwendungszweck angepasster Formulierung, im ungefüllten oder gefüllten Zustand, als Anstrichmitel, Lacke, wie Sinterpulverlacke, als Pressmassen, Tauchharze, Giessharze, Spritzgussformulierungen, Imprägnierharze und Klebmittel, als Werkzweugharze, Laminierharze, Dichtungs- und Spachtelmassen, Bodenbelagsmassen und Bindemittel für mineralische Aggregate verwendet werden.

Die mit den erfindungsgemässen Verbindungen der Formel I hergestellten ausgehärteten Produkte zeichnen sich durch seht gute thermische und mechanische Eigenschaften aus. Sie weisen z.B. hohe Wärmeformbeständigkeit und hohe Hitzebeständigkeit auf, bei gleichzeitiger hoher Biege- bzw. Zugscherfestigkeit. Diese ausgezeichenten Eigenschaften werden auch nach längerer Lagerung in Wasser nicht wesentlich beeinträchtigt. Weiterhin zeichnen sich die ausgehärteten Produkte durch sehr gute chemische Beständigkeit aus.

Bei aliphatischen und insbesondere aromatischen Tetraglycidyloxydiketonen der Formel I ist die hohe Wärmeformbeständigkeit und Biegefestigkeit besonders ausgeprägt, während sich entsprechende aromatische und insbesondere aliphatische Diglycidyloxydiketone der Formel I besonders durch hohe Zugscherfestigkeit auszeichnen.

Die Wahl geeigneter erfindungsgemässen Verbindungen hängt von den gewünschten Eigenschaften der ausgehärteten Produkte und den spezifischen Anwendungen ab.

Die Herstellung sowie die Eigenschaften und Anwendung der erfindungsgemässen Verbindungen sind in den folgenden Beispielen beschrieben.

## Herstellungsbeispiele

### Beispiel 1

4,4'-Diglycidyloxybenzil

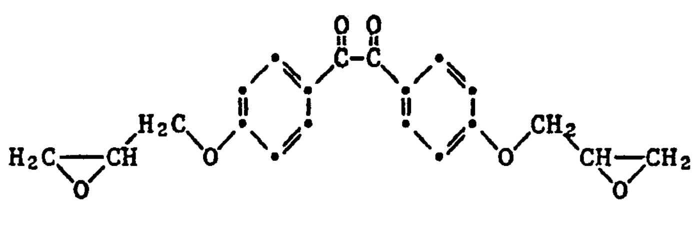

In einem 200 ml Sulfierkolben mit Thermometer, Kühler, Tropftrichter, Rührer und Wasserabscheider werden 6,0 g (0,0248 Mol) 4,4'-Dihydroxybenzil, 34,8 g (0,3752 Mol) Epichlorhydrin und 0.47 g 50%ige wässrige Tetramethylammoniumchloridlösung vorgelegt und auf 105—110°C erwärmt. Nach 4 Std. Reaktionszeit wird das Reaktionsprodukt auf 60°C abgekühlt, und unter gleichzeitigem Wasserauskreisen werden 4,74 g (0,059 Mol) 50%ige wässrige Natriumhydroxidlösung hinzugetropft. Das ausgefallene Kochsalz wird abfiltriert, mit Chloroform gewaschen und das Filtrat eingeengt. Nach Umkristallisation aus Aceton resultieren 5,70 g (64,92 % d.Th.) 4,4'-Diglycidyloxybenzil vom Smp. 120—130°C.

IR (KBr): 3400, 2940, 1640, 1610, 1250 $cm^{-1}$.

NMR ($CDCl_3$): 2,6—3,0 m, 4H (*$CH_2$*—CH—); 3,2—3,5 m 2H (*CH*—$CH_2$); 3,8—4,5 m, 4H (—$OCH_2$—); 6,8—7,7 dd, 8H (arom H).

Epoxidgehalt (Titration mit 0,1 N $HClO_4$): 4,71 Mol/kg (83,58 % d.TH.)

Beispiel 2

1,4-Bis(4'-glycidyloxybenzoyl)benzol

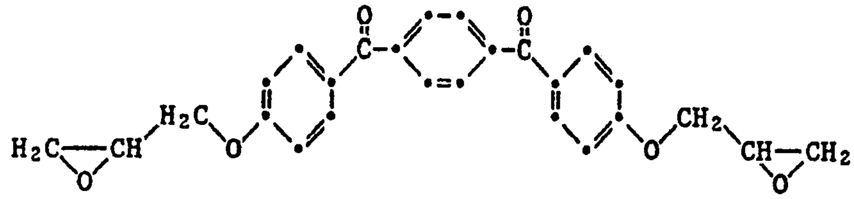

In einem 200 ml Sulfierkolben mit Rührer, Thermometer, Kühler und Wasserabscheider werden 5,0 g (0,0157 Mol) 1,4-Bis(4'-hydroxybenzoyl)benzol, 22,0 g (0,236 Mol) Epichlorhydrin und 0,3 g 50%ige wässrige Tetramethylammoniumchloridlösung vorgelegt und während 4 Std. auf 105—110°C erwärmt. Nach dem Abkühlen auf 60°C werden 3,0 g 50%ige wässrige Natronlauge tropfenweise hinzugefügt unter gleichzeitigem Abscheiden von Wasser. Das Reaktionsgemisch wird filtriert, und das Filtrat wird am Vakuum eingeengt, wobei das 1,4-Bis(4'-glycidyloxybenzoyl)benzol auskristallisiert. Nach dem Trocknen werden 3,14 g (46,4 % d.Th.) leicht beiges 1,4-Bis-(4'-glycidyloxybenzoyl)benzol vom Smp. 199—201°C erhalten.

IR (KBr): 1650, 1600, 1500, 1400, 1250 $cm^{-1}$.

Epoxidgehalt (Titration mit 0,1 N $HClO_4$): 4,646 Mol/kg (93,86 % d.Th.)

Beispiel 3

1,3-Bis(4'-glycidyloxybenzoyl)benzol

In einem 200 ml Sulfierkolben mit Rührer, Kühler, Thermometer und Wasserabscheider werden 10,0 g (0,0314 Mol) 1,3-Bis(4'-hydroxybenzoyl)benzol, 44,0 g (0,475 Mol) Epichlorhydrin und 0,6 g 50%ige wässrige Tetramethylammoniumchloridlösung vorgelegt und auf 110°C erwärmt. Nach 4 Std. Reaktionszeit wird das Reaktionsgemisch auf 60°C abgekühlt, und es werden 6,0 g (0,075 Mol) 50%ige wässrige Natronlauge zugetropft, wobei Natriumchlorid als Festkörper ausscheidet. Nach Beendigung des Wasserabscheidens wird das Reaktionsgemisch filtriert, das Filtrat wird in Epichlorhydrin aufgenommen, mit Wasser gewaschen und über Natriumsulfat getrocknet, filtriert und am Vakuum eingeengt. Es resultieren 13,10 g (97 % d.Th.) eines viskosen Harzes, welches beim Stehenlassen auskristallisiert. Das Rohprodukt wird aus Methylcellosolve/Wasser umkristallisiert, wobei nach dem Trocknen 9,38 g (69,43 % d.Th.) 1,3-Bis(4'-glycidyloxybenzoyl)benzol vom Smp. 88—93°C als praktisch farblose Nadeln isoliert werden.

IR (KBr): 1650, 1600, 1250 $cm^{-1}$.

NMR ($CDCl_3$): 2,5—3,1 m 4H (*$CH_2$*—CH—); 3,3—3,6 m 2H (*CH*—$CH_2$); 4,0—4,5 m 4H (—$OCH_2$—); 6,8—8,0 m 12H (arom. H);

Epoxidgehalt (Titration mit 0,1 N $HClO_4$): 4,26 Mol/kg (91,69 % d/Th.).

Beispiel 4

1,4-Bis(4'-glycidyloxy-3',5'-dimethylbenzoyl)benzol

In einem 200 ml Sulfierkolben mit Rührer, Kühler, Thermometer und Wasserabscheider werden 13,10 g (0,035 Mol) 1,4-Bis(4'-hydroxy-3',5'-dimethylbenzoyl)benzol, 49,0 g (0,52 Mol) Epichlorhydrin und 0,5 g 50%ige wässrige Tetramethylammoniumchloridlösung auf 105—110°C erwärmt. Nach 4 Std. Reaktionszeit wird das Reaktionsgemisch auf 60°C abgekühlt, und tropfenweise werden 12,8 g (0,16 Mol) 50%ige wässrige Natronlauge unter gleichzeitigem Auskreisen von Wasser zugegeben. Das ausgefallene Natriumchlorid wird abfiltriert, das Filtrat mit Epichlorhydrin gewaschen und am Vakuum eingeengt. Der Rückstand (15,0 g) wird in 50 ml Toluol umkristallisiert, filtriert und getrocknet. Es resultieren 11,5 g (67,5 % d.Th.) 1,4-Bis(4'-glycidyloxy-3',5'-dimethylbenzoyl)benzol vom Smp. 164—170°C als praktisch farbloses Pulver.

IR (KBr): 2920, 1640, 1590, 1320, 1210, 1130, 1000 $cm^{-1}$.

NMR ($CDCl_3$): 2,33 s 12H (—$CH_3$); 2,6—3,0 m 4H (—*$CH_2$*—CH—); 3,2—3,6 m 2H (—*CH*—$CH_2$—); 3,6—4,3 m 4H (O—$CH_2$—); 7,0—7,9 m 8H (arom—H).

Epoxidgehalt (Titration mit 0,1 $HClO_4$): 3,91 Mol/kg (95,03 % d.Th.).

Elementaranalyse: $C_{30}H_{30}O_6$ (486,56)

berechnet C 74,06% H 6,22%

gefunden C 74,16% H 6,32%

Beispiel 5

1,4-Bis(4'-glycidyloxybenzoyl)butan

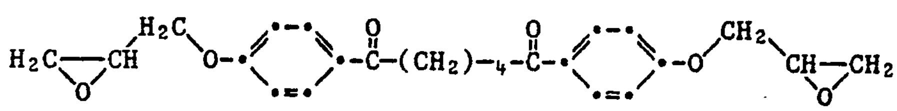

In einem 350 ml Sulfierkolben mit Rührer, Thermometer, Kühler, Tropftrichter und Wasserabscheider werden 16,41 g (0,055 Mol) 1,4-Bis(4'-hydroxybenzoyl)butan, 77,07 g (0,832 Mol) Epichlorhydrin und 1,05 g einer 50%igen wässrigen Lösung von Tetramethylammoniumchlorid suspendiert. Das Gemisch wird während 4 h am Rückfluss erwärmt (112—116°C); anschliessend wird das Reaktionsprodukt auf 60°C abgekühlt und unter langsamen Zutropfen von 10,51 g (0,131 Mol) wässriger Natronlauge wird bei 40°C innerhalb ca. 2,5 Std. das Wasser azeotrop entfernt. Das Reaktionsgemisch wird noch während ca. 16 Std. bei dieser Temperatur gerührt, anschliessend in einen Scheidetrichter übergeführt, mehrmals mit Wasser gewaschen, getrocknet und eingeengt, wobei ein semikristallines Produkt isoliert wird. Nach Umkristallisation aus Aceton werden 11,00 g (48,72% d.Th.) 1,4-Bis(4'-glycidyloxybenzoyl)butan vom Smp. 126—130°C erhalten.

IR (KBr): 3500—3300, 3100—2940, 1670, 1600, 1510, 1360, 1260, 1170, 1030, 970, 920, 830 $cm^{-1}$.

NMR ($d_6$-DMSO): 1,5—1,8 m 4H ($CH_2$-aliph.); 2,4—4,6 m 14H (—$CH_2CO$— und glycidyl-H); 6,8—7,8 dd (J=8Hz), (arom H).

Epoxidgehalt (Titration mit 0,1 N $HClO_4$): 4,835 Mol/kg (99,25 % d.Th.).

## Beispiel 6

1,8-Bis(4'-glycidyloxybenzoyl)octan

In einem 200 ml Sulfierkolben mit Kühler, Thermometer, Rührer, Tropftrichter und Wasserabscheider werden 17,72 g (0,05 Mol) 1,8-Bis(4'-hydroxybenzoyl)octan, 70,03 g (0,756 Mol) Epichlorhydrin und 0,96 g 50%ige wässrige Tetramethylammoniumchloridlösung vorgelegt und auf 105—110°C erwärmt. Nach 4 Std. wird das Reaktionsgemisch auf 60°C abgekühlt und unter gleichzeitigem Wasserabscheiden werden 9,55 g (0,118 Mol) 50%ige wässrige Natriumhydroxidlösung hinzugetropft. Das Reaktionsgemisch wird in Epichlorhydrin aufgenommen, mit Wasser gewaschen, getrocknet und eingeengt. Es resultieren 21,73 g (93,14% d.Th.) farbloses 1,8-Bis(4'-glycidyloxybenzoyl)octan vom Smp. 118—119°C.

IR (KBr): 3500, 2920, 2840, 1680, 1600, 1230 $cm^{-1}$.

NMR ($CDCl_3$): 1,0—2,0 m 12H (—$CH_2$)$_6$; 2,7—3,0 m 8H

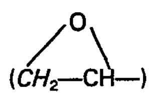 und 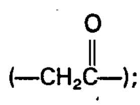

3,0—3,5 m 2H (—*CH*—$CH_2$); 3,7—4,5 m 4H ($CH_2O$); 6,7—7,8 dd 8H (arom. H).

Epoxidgehalt (Titration mit 0,1 N $HClO_4$): 3,988 Mol/kg (93,03 % d.Th.).

## Beispiel 7

1,4-Bis(2',4'-diglycidyloxybenzoyl)benzol

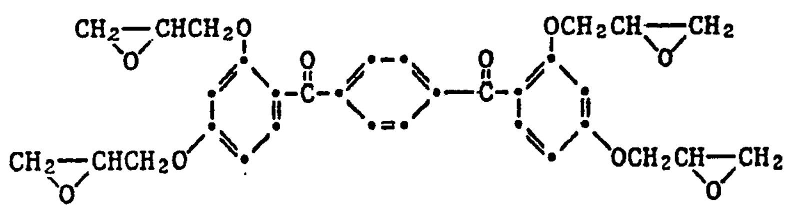

In einem 200 ml Sulfierkolben mit Rührer, Kühler, Thermometer und Wasserabscheider werden 8,0 g (0,0228 Mol) 1,4-Bis(2,',4'-dihydroxybenzoyl)benzol, 31,7 g (o,343 Mol) Epichlorhydrin und 0,4 g 50%ige wässrige Tetramethylammoniumchloridlösung vorgelegt und während 4 Std. auf 105—110°C erwärmt. Anschliessend wird das Reaktionsgemisch auf 60°C abgekühlt, und unter gleichzeitigem Auskreisen von Wasser werden 8,3 g (0,104 Mol) 50%ige wässrige Natronlauge hinzugetropft. Der Rückstand wird in Epichlorhydrin aufgenommen, das ausgefallene Kochsalz wird filtriert und das Filtrat wird am Vakuum eingeengt. Es resultieren 14,7 g eines braunen Oels, welches an Kieselgel (Toluol:Ethanol 90:10) filtriert wird, wobei 3,22 g (24,6 % d.Th.) 1,4-Bis(2',4'-diglycidyloxybenzoyl)benzol vom Smp. 128—133°C erhalten werden.

IR (KBr): 3060, 2860, 1640, 160, 1250 $cm^{-1}$.

NMR ($d_6$-DMSO): 2,4—2,6 m 8H (*$CH_2$*CH—); 2,8—3,1 m 4H (—*CH*—$CH_2$); 3,7—4,5 m 8H (—$OCH_2$); 6,6—7,8 m 10H (arom. H).

Epoxidgehalt (Titration mit 0,1 N $HClO_4$): 5,81 Mol/kg (83,5 % d.Th.).

## Beispiel 8

1,3-Bis(2',4'-diglycidyloxybenzoyl)benzol

In einem 1,5 l Sulfierkolben mit Rührer, Kühler, Thermometer und Wasserabscheider werden 150 g (0,427 Mol) 1,3-Bis(2',4'-dihydroxybenzoyl)benzol, 59,4 g (0,642 Mol) Epichlorhydrin und 8,0 g 50%ige wässrige Tetramethylammoniumchloridlösung vorgelegt und auf 105—110°C erwärmt. Nach 4 Std. Reaktionszeit wird das Reaktionsgemisch auf 60°C abgekühlt, und unter gleichzeitigem Wasserauskreisen

werden 155,8 g (1,95 Mol) 50%ige wässrige Natronlauge hinzugetropft, wobei fein verteiltes Kochsalz ausfällt. Das Reaktionsgemisch wird filtriert, das Filtrat wird mit Wasser gewaschen, über $Na_2SO_4$ getrocknet und im Vakuum eingeengt. Es resultieren 242,7 g (98,9 % d.Th.) 1,3-Bis(2',4'-diglycidyloxybenzoyl)benzol als oranges, viskoses Oel.

IR (KBr): 3000, 2860, 1650, 1600, 1250 $cm^{-1}$.

NMR ($CDCl_3$): 2,5—3,0 m 8H (—$CH_2$—CH—); 3,2—3,5 m 4H (—$CH_2$—*CH*—); 3,5—4,3 m 8H (—O—$CH_2$—); 6,5—7,8 m 10H (arom. H).

Epoxidgehalt (Titration mit 0,1 N $HClO_4$): 5,589 Mol/kg (80,3 % d.Th.).

Beispiel 9

1,4-Bis(2',4'-diglycidyloxybenzoyl)butan

In einem 350 ml Sulfierkolben mit Rührer, Thermometer, Kühler, Tropftrichter und Wasserabscheider werden 18,16 g (0,055 Mol) 1,4-Bis(2',4'-dihydroxybenzoyl)butan, 86,84 g (0,938 Mol) Epichlorhydrin und 0,97 g 50%ige wässrige Tetramethylammoniumchloridlösung vorgelegt und auf 105—110°C erwärmt. Nach 4 Std. Reaktionszeit wird das Reaktionsgemisch auf 60°C abgekühlt; anschliessend werden unter gleichzeitigem Wasserauskreisen 20,21 g (0,253 Mol) 50% ige wässrige Natriumhydroxidlösung hinzugetropft. Die entstehende Suspension wird mit Wasser versetzt, und die organische Phase wird mit Wasser gewaschen, über Natriumsulfat getrocknet und eingeengt. Es resultieren 28,62 g (93,84 % d.Th.) eines roten viskosen Harzes.

IR (Film): 3500, 3030, 3000, 2820, 1660, 1250 $cm^{-1}$.

NMR ($d_6$-DMSO): 1,2—1,6 s br 4H (—$CH_2$— aliph.); 2,0—4,5 m 24H (Glycidyl-H und —$CH_2$CO—); 6,5—7,3 m 6H (arom. H).

Epoxidgehalt (Titration mit 0,1 N $HClO_4$): 5,307 Mol/kg (73,58 % d.Th.).

Verwendungsbeispiele

Beispiel I—VIII

Je 100 Gewichtsteile des entsprechenden Glycidyloxydiketons werden jeweils mit der in Tabelle 1 angegebenen Menge Bis(4-aminophenyl)methan als Härter vermischt. Von diesen Mischungen werden einerseits die Reaktivität mittels de Gelierzeit sowie der Differential-Thermoanalyse (DTA) und andererseits die Glasumwandlungstemperatur (Tg) und die Zugscherfestigkeit der ausgehärteten Produkte gemessen.

In den Beispielen werden die folgenden Verbindungen verwendet:

I: 1,4-Bis(4'-glycidyloxybenzoyl)benzol, (gemäss Bsp. 2).
II: 1,3-Bis(4'-glycidyloxybenzoyl)benzol, (gemäss Bsp. 3).
III: 1,4-Bis(4'-glycidyloxy-3',5'-dimethylbenzoyl)benzol, (gemäss Bsp. 4).
IV: 1,4-Bis(4'-glycidyloxybenzoyl)butan, (gemäss Bsp. 5).
V: 1,8-Bis(4'-glycidyloxybenzoyl)octan, (gemäss Bsp. 6).
VI: 1,4-Bis(2',4'-diglycidyloxybenzoyl)benzol, (gemäss Bsp. 7).
VII: 1,3-Bis(2',4'-diglycidyloxybenzoyl)benzol, (gemäss Bsp. 8).
VIII: 1,4-Bis(2',4'-diglycidyloxybenzoyl)butan, (gemäss Bsp. 9).

Tabelle 1

| Beispiel | I | II | III | IV | V | VI | VII | VIII |
|---|---|---|---|---|---|---|---|---|
| Härter (Gewichtsteile) | 21,6 | 21,1 | 19,4 | 24,0 | 19,8 | 28,8 | 25,6 | 26,3 |
| Gelierzeit 140°C | nicht geschmolzen | 5'55" | 19' | 5'20" | 10' | 2' | 1'50" | 1'15" |
| 160°C | 3'10" | 4'35" | 18'40" | 4'15" | 6'15" | 1'25" | 1'25" | 1'05" |
| 180°C | 55" | 1'15" | 4'50" | 1'15" | 7'50" | 30" | 30" | 20" |
| DTA[f)] Smp. (°C) | 91 | 60+74 | - | 81+117 | 81+112 | 71 | - | - |
| $T_B$ (°C) | 96 | 85 | 115 | 89 | ~115 | 81 | 58 | 61 |
| $T^{RG}$ max (°C) | 139 | 142 | 168 | 137 | 147 | 127 | 127 | 126 |
| $T^E$ (°C) | 206 | 238 | 245 | 247 | 241 | 241 | 199 | 206 |
| $\Delta H$[a)] (kJ) | 72,4 | 103,6 | 81,3 | 101,7[d)] | 106,3[d)] | 72,4 | 98,4 | 105,5 |
| Tg (°C) | - | 162[b)] | 166[b)] | 122[b)] | 113[b)] | 169[b)] | 164[b)] | 168[b)] |
| | 166[c)] | 163[c)] | 198[c)] | 162[c)] | 107[c)] | 209[c)] | 208[c)] | 210[c)e)] |
| Zugscherfestigkeit (N/mm²) (DIN 53283) | - | 10,0[b)] | 11,8[b)] | 14,5[b)] | 21,9[b)] | 6,1[b)] | 3,1[b)] | 4,5[b)] |
| | 8,6[c)] | 9,3[c)] | 10,5[c)] | 11,9[c)] | 22,1[c)] | 6,0[c)] | 4,7[c)] | 2,5[c)] |

a) Reaktionsenthalpie pro Epoxidäquivalent
b) Härtungszyklus: 4 h bei 80°C vorgeliert, 8 h bei 140°C
c) Härtungszyklus: 4 h bei 80°C vorgeliert, 8 h bei 140°C + 6 h bei 180°C
d) extrapoliert
e) Mit TMS (Thermomechanical Scanning Calorimeter) bestimmt
f) auf Mettler TA 3000, Aufheizgeschwindigkeit 4°C/min.

An den ausgehärteten Produkten gemäss den Beispielen II—VIII wird auch die chemische Beständigkeit nach DIN 53 230 bestimmt. In 5N $H_2SO_4$, 5N NaOH, Wasser, Aceton und Chlorbenzol zeichen sich die nach dem Härtungszyklus (c) ausgehärteten Produkte der Beispiele II—VIII durchwegs durch ausgezeichnete Prüfungswerte "0" aus. Dasselbe trifft für die nach dem Härtungszyklus (b) ausgehärteten Produkte der Beispiele II, IV, V und VIII zu, während die entsprechenden nicht vollständig ausgehärteten Produkte der Beispiele III, VI und VII in 5N $H_2SO_4$ und/oder in Aceton bzw. in Chlorbenzol etwas weniger gute Prüfungswerte "1" aufweisen.

Beispiel IX

100 Gewichtsteile 1,3-Bis-(2',4'-diglycidyloxybenzoyl)benzol (hergestellt nach Beispiel 8) werden mit 27,7 Gewichtsteilen Bis(4-aminophenyl)methan vermischt und (A) 4 h bei 100°C und 8 h bei 140°C, bzw. (B) 4 h bei 100°C, 8 h bei 140°C und 6 h bei 180°C gehärtet. Es werden die folgenden Eigenschaften ermittelt:

| | A | B | |
|---|---|---|---|
| Schlagbiegefestigkeit (VSM 77105) | 17 | 12 | kJ/m² |
| Biegefestigkeit (ISO 178) | 180 | 154 | N/mm² |
| Randfaserdehnung (ISO 178) | 6,9 | 4,1 | % |
| Glasumwandlungstemperatur | 174 | 202 | °C |
| Wärmeformbestandigkeit (ISO 75) | 163 | 192 | °C |
| Wasseraufnahme — nach 4 Tagen bei 25°C | 0,41 | 0,40 | Gew.% |
| 1 Stunde Kochwasser | 0,42 | 0,34 | Gew.% |
| 100 Stunden Kochwasser | 5,2 | 0,34 | Gew.% |
| Dielektrischer Verlustfaktor tanδ | | | |
| (DIN 53483) >1% oberhalb | 55 | 40 | °C |
| >5% oberhalb | 104 | 90 | °C |
| Dielektr. Zahl ε bei 23°C (DIN 53483) | 4,9 | 4,7 | |
| Spez. Durchgangswiderstand bei 23°C (DIN 53482) | $2{,}3\cdot10^{15}$ | $1{,}9\cdot10^{15}$ | Ohm cm |

Beispiel X

100 Gewichtsteile 1,3-Bis(2',4'-diglycidyloxybenzoyl)benzol (hergestellt nach Beispiel 8) werden mit 33,1 Gewichtsteilen Bis(4-aminophenyl)sulfon vermischt, und es werden die folgenden Eigenschaften des Gemisches ermittelt:

Differential Scanning Calorimetry (DSC) auf Mettler TA 3000

| | | |
|---|---|---|
| (25°C → 300°C, 4°C/min) | $T_1$ | 90°C |
| | $T_2$ | 171°C |
| | $T_3$ | 254°C |
| | Single T 50% | 178,4°C |
| | ΔH | 336,2 J/g |
| | Tg | 196°C |
| Gelierzeit 120°C (Platte) | | 36′ |
| 140°C | | 16′30″ |
| 160°C | | 8′30″ |
| 180°C | | 4′30″ |

Beispiel XI

Das im Beispiel X beschriebene Harz/-Härter-Gemisch wird (A) 2 h bei 180°C und (B) 4 h bei 200°C gehärtet. Es werden die folgenden Eigenschaften bestimmt:

| | | A | B |
|---|---|---|---|
| Glasumwandlungstemperatur | DSC | 216 | 217°C |
| (thermisch-mechanische Analyse) | TMA | 216 | 222°C |
| ("torsional braid analyzer") | TBA | 206 | 231°C |
| $^1/_p{}^2$ (Steifheit) | | 0,34 | 0,28 |
| Glasumwandlungstemperatur TBA nach Lagerung, 14 Tage in $H_2O$ bei 31°C | | 241 | 246°C |
| $^1/_p{}^2$ (Steifheit) | | 0,14 | 0,12 |
| % Aenderung | | −59 | −57 |
| Thermogravimetrische Analyse (TGA) (RT → 900°C) 50% Gewichtsverlust | | — | 614°C |
| Lacktechnische Eigenschaften Erichsentiefe (DIN 53156) | | 1,8 | 1,3 mm |
| Schlagerichsen | | <10 | <10 cm·kg |
| MEK-Reibtest (DIN 53 230) | | 0 | 0 |

**Patentansprüche**

1. Verbindungen der Formel I

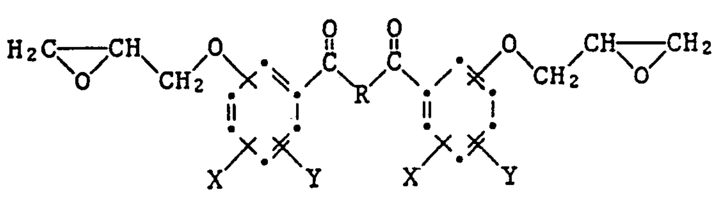

(I),

worin die Substituenten X unabhängig voneinander Wasserstoff, Alkyl mit 1 bis 6 C-Atomen, Glycidyloxy oder Alkoxy mit 1 bis 6 C-Atomen bedeuten, die Substituenten Y unabhängig voneinander Wasserstoff oder Alkyl mit 1 bis 6 C-Atomen bedeuten und R für eine direkte Bindung, eine Gruppe der Formel $-C_mH_{2m}-$, $-C_nH_{2n-2}-$ oder $-C_nH_{2n-4}-$ mit m=1—12 und n=2—12, einen zweiwertigen, gegebenenfalls ungesättigten, cycloaliphatischen Rest mit bis zu 14 C-Atomen, einen zweiwertigen araliphatischen Rest mit bis zu 14 C-Atomen, einen zweiwertigen $C_6$—$C_{12}$ aromatischen Rest oder eine Gruppe Der Formel II

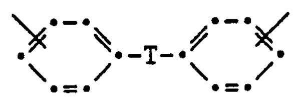

(II),

worin T eine direkte Bindung, Methylen, Isopropyliden, O, S, NH, CO oder $SO_2$ bedeutet, steht.

2. Verbindungen der Formel I nach Anspruch 1, worin die Substituenten X sowie die Substituenten Y jeweils gleich sind, die Substituenten X und Y sowie die Glycidyloxygruppen jeweils in der gleichen Stellung in Bezug auf die Carbonylgruppe sind und R für eine Gruppe der Formel $-C_mH_{2m}-$ mit m=1 bis 8 oder Phenylen steht.

3. Verbindungen der Formel I nach Anspruch 2, worin die Substituenten X jeweils Wasserstoff, Alkyl mit 1 bis 4 C-Atomen oder Alkoxy mit 1 bis 4 C-Atomen und die Substituenten Y jeweils Wasserstoff oder Alkyl mit 1 bis 4 C-Atomen bedeuten, und die Glycidyloxygruppen jeweils in der para-Stellung zu den Carbonylgruppen gebunden sind.

4. Verbindungen der Formel I nach Anspruch 3, worin die Substituenten X und Y jeweils gleich sind und Methyl oder insbesondere Wasserstoff bedeuten und R für 1,4-Phenylen, 1,3-Phenylen, 1,4-Butylen oder 1,8-Octylen steht.

5. Verbindungen der Formel I nach Anspruch 2, worin die Substituenten X jeweils eine Glycidyloxygruppe und die Substituenten Y jeweils Wasserstoff oder eine Alkylgruppe mit 1 bis 4 C-Atomen darstellen, und die Glycidyloxygruppen jeweils in der ortho- und der para-Stellung zu den Carbonylgruppen gebunden sind.

6. Verbindungen der Formel I nach Anspruch 5, worin die Substituenten Y jeweils Wasserstoff sind und R für 1,4-Butylen und insbesondere für 1,3-Phenylen oder 1,4-Phenylen steht.

7. Verfahren zur Herstellung von Verbindungen der Formel I nach Anspruch 1, dadurch gekennzeichnet, dass man eine Verbindung der Formel III

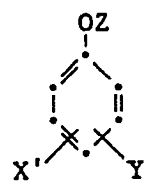

(III)

oder ein Gemisch verschiedener Verbindungen der Formel III, worin Z Wasserstoff oder Alkyl mit 1 bis 6 C-Atomen und X' Wasserstoff, Alkyl mit 1 bis 6 C-Atomen, Hydroxy oder Alkoxy mit 1 bis 6 C-Atomen darstellen, und für Y das in Anspruch 1 Angegebene gilt, in Anwesenheit eines Friedel-Crafts-Katalysators mit einer Verbindung der Formel IV

Cl—CO—R—CO—Cl (IV)

worin R die im Anspruch 1 angegebene Bedeutung hat, in eine Verbindung der Formel V

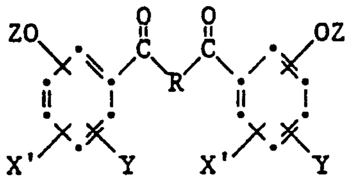

(V)

überführt, die Verbindung der Formel V gegebenenfalls O-dealkyliert und anschliessend mit einem Epihalogenhydrin umsetzt, wobei vor oder nach der Reaktion mit dem Epihalogenhydrin gegebenenfalls eine O-Alkylierung mit einem $C_{1-6}$-Alkylhalogenid durchgeführt werden kann.

8. Verbindungen der Formel V

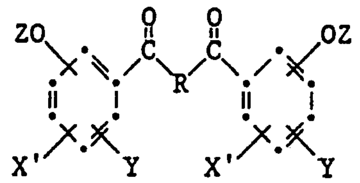

(V)

worin die Substituenten X′ unabhängig voneinander Wasserstoff, Alkyl mit 1 bis 6 C-Atomen, Hydroxy oder Alkoxy mit 1 bis 6 C-Atomen bedeuten, die Substituenten Y unabhängig voneinander Wasserstoff oder Alkyl mit 1 bis 6 C-Atomen bedeuten, die Gruppen Z unabhängig voneinander Wasserstoff oder Alkyl mit 1 bis 6 C-Atomen bedeuten und R für eine direkte Bindung, eine Gruppe der Formel $-C_mH_{2m}-$, $-C_nH_{2n-2}-$ oder $-C_nH_{2n-4}-$ mit m=1—12 und n=2—12, einen zweiwertigen, gegebenenfalls ungesättigten cycloaliphatischen Rest mit bis zu 14 C-Atomen, einen zweiwertigen $C_6$—$C_{12}$ aromatischen Rest oder eine Gruppe der Formel II

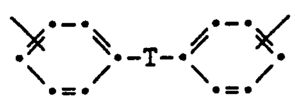

(II),

worin T eine direkte Bindung, Methylen, Isopropyliden, O, S, NH, CO oder $SO_2$ bedeutet, steht, mit der Massgabe, dass R nicht 1,3-Phenylen oder $C_1$—$C_5$-Alkyliden ist.

9. Verwendung von Verbindungen der Formel I nach Anspruch 1 zur Herstellung von vernetzten Produkten.

**Revendications**

1. Composés répondant à la formule I:

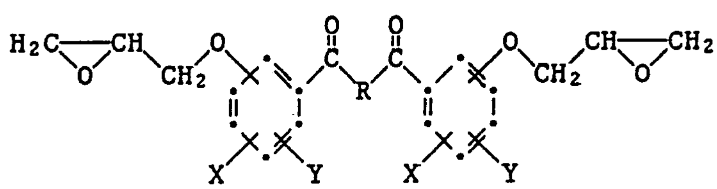

(I),

dans laquelle:

les X représentent chacun, indépendamment l'un de l'autre, l'hydrogène, un alkyle contenant de 1 à 6 atomes de carbone, un glycidyloxy ou un alcoxy contenant de 1 à 6 atomes de carbone,

les Y représentent chacun, indépendamment l'un de l'autre, l'hydrogène ou un alkyle contenant de 1 à 6 atomes de carbone, et

R représente une liaison directe, un radical répondant à l'une des formules:

$$-C_mH_{2m}-,\ -C_nH_{2n-2}-\ \text{et}\ -C_nH_{2n-4}-$$

dans lesquelles m désigne un nombre de 1 à 12 et n un nombre de 2 à 12, un radical cycloaliphatique bivalent, éventuellement insaturé, qui contient au plus 14 atomes de carbone, un radical araliphatique bivalent qui contient au plus 14 atomes de carbone, un radical aromatique bivalent en $C_6$—$C_{12}$ ou un radical de formule II:

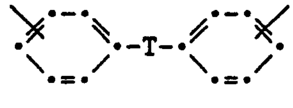

(II),

dans lequel T représente une liaison directe, un méthylène, un isopropylidène, O, S, NH, CO ou $SO_2$.

2. Composés de formule I selon la revendication 1 dans lesquels les symboles X ont la même signification, les symboles Y aussi, les radicaux X et Y ainsi que les radicaux glycidyloxy se trouvent chacun en la même position relativement au radical carbonyle correspondant, et R représente un radical de formule $-C_mH_{2m}-$ (dans lequel m désigne un nombre de 1 à 8) ou un radical phénylène.

3. Composés de formule I selon la revendication 2 dans lesquels les symboles X représentent chacun l'hydrogène, un alkyle en $C_1$—$C_4$ ou un alcoxy en $C_1$—$C_4$, les symboles Y représentent chacun l'hydrogène ou un alkyle en $C_1$—$C_4$, et les radicaux glycidyloxy se trouvent chacun en la position para relativement au radical carbonyle correspondant.

4. Composés de formule I selon la revendication 3 dans lesquels les symboles X ont la même signification et représentent chacun un méthyle ou, mieux, l'hydrogène, les symboles Y ont la même signification et représentent chacun un méthyle ou, mieux, l'hydrogène, et R représentent un radical phénylène-1,4, phénylène-1,3, butylène-1,4 ou octylène-1,8.

5. Composés de formule I selon la revendication 2 dans lesquels les X représentent chacun un radical glycidyloxy, les Y représentent chacun l'hydrogène ou un alkyle en $C_1$—$C_4$, et les radicaux glycidyloxy se trouvent chacun en position ortho ou para relativement au radical carbonyle correspondant.

6. Composés de formule I selon la revendication 5 dans lesquels les Y représentent chacun l'hydrogène et R représente un radical butylène-1,4 ou, mieux, phénylène-1,3 ou phénylène-1,4.

7. Procédé pour préparer des composés de formule I selon la revendication 1, procédé caractérisé en ce qu'on transforme un composé répondant à la formule III (ou un mélange de tels composés):

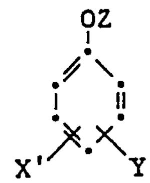

(III)

formule dans laquelle Z représente l'hydrogène ou un alkyle contenant de 1 à 6 atomes de carbone, X' représente l'hydrogène, un alkyle contenant de 1 à 6 atomes de carbone, un hydroxy ou un alcoxy contenant de 1 à 6 atomes de carbone, et Y a la signification qui lui a été donnée à la revendication 1, en présence d'un catalyseur de Friedel-Crafts, avec un composé répondant à la formule IV:

$$Cl—CO—R—CO—Cl \qquad (IV)$$

dans laquelle R a la signification qui lui a été donnée à la revendication 1, en un composé de formule V:

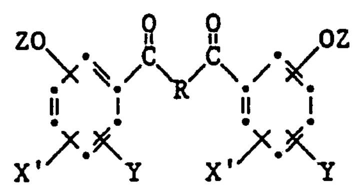

(V)

on désalkyle éventuellement à l'oxygène le composé de formule V, on la fait ensuite réagir avec une épihalogénhydrine, et, avant ou après la réaction avec l'épihalogénhydrine, on effectue éventuellement une alkylation à l'oxygène au moyen d'un halogénure d'alkyle en $C_1$—$C_6$.

8. Composés répondant à la formule V:

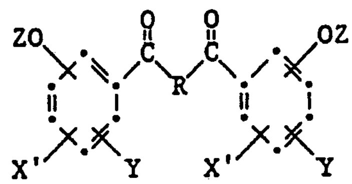

(V)

dans laquelle:

les X' représentent chacun, indépendamment l'un de l'autre, l'hydrogène, un alkyle contenant de 1 à 6 atomes de carbone, un hydroxy ou un alcoxy contenant de 1 à 6 atomes de carbone,

les Y représentent chacun, indépendamment l'un de l'autre, l'hydrogène ou un alkyle contenant de 1 à 6 atomes de carbone,

les Z représentent chacun, indépendamment l'un de l'autre, l'hydrogène ou un alkyle contenant de 1 à 6 atomes de carbone et

R représente une liaison directe, un radical répondant à l'une des formules $—C_mH_{2m}—$, $—C_nH_{2n-2}—$ et $—C_nH_{2n-4}—$ dans lesquelles m désigne un nombre de 1 à 12 et n un nombre de 2 à 12, un radical cycloaliphatique bivalent, éventuellement insaturé, qui contient au plus 14 atomes de carbone, un radical aromatique bivalent en $C_6$—$C_{12}$ ou un radical répondant à la formule II:

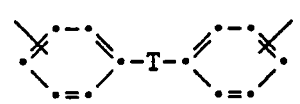

(II),

dans laquelle T représente une liaison directe, un méthylène, un isopropylidène, O, S, NH, CO ou $SO_2$, avec la condition que R ne représente ni un radical phénylène-1,3 ni un radical alkylidène en $C_1$—$C_5$.

9. Application de composés de formule I selon la revendication 1 à la fabrication de produits durcis.

**Claims**

1. A compound of the formula I

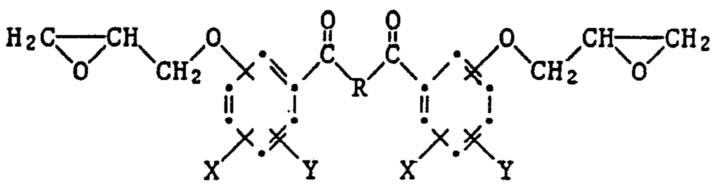

(I)

in which the substituents X independently of one another are hydrogen, alkyl having 1 to 6 C atoms, glycidyloxy or alkoxy having 1 to 6 C atoms, the substituents Y independently of one another are hydrogen or alkyl having 1 to 6 C atoms and R is a direct bond, a group of the formula $—C_mH_{2m}—$, $—C_nH_{2n-2}—$ or $—C_nH_{2n-4}—$ in which m=1—12 and n=2—12, a divalent, saturated or unsaturated cycloaliphatic radical having up to 14 C atoms, a divalent araliphatic radical having up to 14 C atoms, a divalent $C_6—C_{12}$ aromatic radical or a group of the formula II

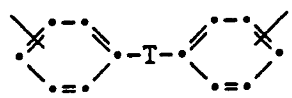

(II)

in which T is a direct bond, methylene, isopropylidene, O, S, NH, CO or $SO_2$.

2. A compound of the formula I according to claim 1, in which the substituents X and the substituents Y are each identical, the substituents X and Y and the glycidyloxy groups are each in the same substituents X and the substituents Y are each identical, the substituents X and Y and the glycidyloxy groups are each in the same position relative to the carbonyl group and R is a group of the formula $—C_mH_{2m}—$ in which m=1 to 8, or phenylene.

3. A compound of the formula I according to claim 2, in which the substituents X are each hydrogen, alkyl having 1 to 4 C atoms or alkoxy having 1 to 4 C atoms and the substituents Y are each hydrogen or alkyl having 1 to 4 C atoms and the glycidyloxy groups are each attached in the para-position relative to the carbonyl groups.

4. A compound of the formula I according to claim 3, in which the substituents X and Y are in each case identical and are methyl or, in particular, hydrogen and R is 1,4-phenylene, 1,3-phenylene, 1,4-butylene or 1,8-octylene.

5. A compound of the formula I according to claim 2, in which the substituents X are each a glycidyloxy group and the substituents Y are each hydrogen or an alkyl group having 1 to 4 C atoms and the glycidyloxy groups are each attached in the ortho-position and the para-position relative to the carbonyl groups.

6. A compound of the formula I according to claim 5, in which the substituents Y are each hydrogen and R is 1,4-butylene and, in particular, 1,3-phenylene or 1,4-phenylene.

7. A process for the preparation of compounds of the formula I according to claim 1, which comprises converting a compound of the formula III

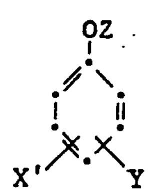

(III)

or a mixture of different compounds of the formula III in which Z is hydrogen or alkyl having 1 to 6 C atoms and X' is hydrogen, alkyl having 1 to 6 C atoms, hydroxyl or alkoxy having 1 to 6 C atoms and Y is as defined in claim 1, in the presence of a Friedel-Crafts catalyst and by means of a compound of the formula IV

$$Cl—CO—R—CO—Cl \qquad (IV)$$

in which R is as defined in claim 1, into a compound of the formula V

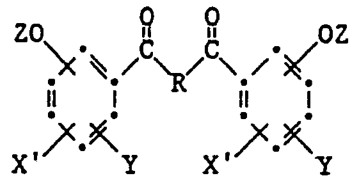

(V)

if appropriate O-dealkylating the compound of the formula V and then reacting the product with an epihalogenohydrin, it being possible, if desired, to carry out an O-alkylation by means of a $C_1$—$C_6$ alkyl halide before or after the reaction with the epihalogenohydrin.

8. A compound of the formula V

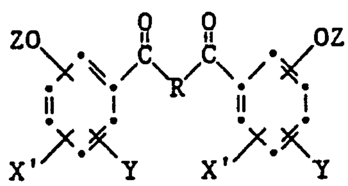

(V)

in which the substituents X' independently of one another are hydrogen, alkyl having 1 to 6 C atoms, hydroxyl or alkoxy having 1 to 6 C atoms, the substituents Y independently of one another are hydrogen or alkyl having 1 to 6 C atoms, the groups Z independently of one another are hydrogen or alkyl having 1 to 6 C atoms and R is a direct bond, a group of the formula $—C_mH_{2m}—$, $—C_nH_{2n-2}—$ or $—C_nH_{2n-4}—$ in which m=1—12 and n=2—12, a divalent, saturated or unsaturated cycloaliphatic radical having up to 14 C atoms, a divalent $C_6$—$C_{12}$ aromatic radical or a group of the formula II

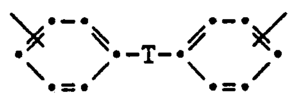

(II)

in which T is a direct bond, methylene, isopropylidene, O, S, NH, CO or $SO_2$, with the proviso that R is not 1,3-phenylene or $C_1$—$C_5$ alkylidene.

9. The use of compounds of the formula I according to claim 1 for the preparation of crosslinked products.